# EUROPEAN PATENT APPLICATION

(11) **EP 4 840 324 A1**
(43) Date of publication of application: **18.11.2026**
(21) Application number: 25739144.1
(22) Date of filing: 10.01.2025
(51) Int. Cl.: A23L 33/105, A61K 36/31, A61P 3/04, A23K 10/30

(54) **COMPOSITION FOR PREVENTING, AMELIORATING OR TREATING OBESITY COMPRISING WASABI LEAF EXTRACT AS ACTIVE INGREDIENT**

(30) Priority: 11.01.2024 KR 20240004918
(71) Applicant: Nutracore Co., Ltd., Suwon-si, Gyeonggi-do 16514 (KR)
(72) Inventor: CHOI, Beom Rak, Suwon-si, Gyeonggi-do 16344 (KR); KU, Sae-Kwang, Daegu 42281 (KR); PARK, Hye-Rim, Hwaseong-si, Gyeonggi-do 18488 (KR)
(74) Representative: Dehns
(86) International application number: PCT/KR2025/000643
(87) International publication number: WO 2025/150984

(57) **Abstract**

The present invention relates to a composition for treating obesity, comprising a wasabi leaf extract, which is a natural extract, as an active ingredient. The present invention relates to a composition for preventing, ameliorating or treating obesity, comprising a wasabi leaf extract, wherein the wasabi leaf extract includes 0.8-1.6 mg/g of isovitexin and 4.8-9.6 mg/g of sinigrin, and the weight ratio of isovitexin to sinigrin in the composition is 1:1-1:6 (isovitexin: sinigrin). The composition of the present invention exhibits the effects of reducing body weight, body fat, and abdominal fat without causing appetite reduction.

## Description

### [Technical Field]

This application claims priority to and the benefit of Korean Patent Application No. 10-2024-0004918, filed on January 11, 2024, the disclosure of which is incorporated herein by reference in its entirety.

The present invention relates to a composition for preventing, improving, or treating obesity, comprising a wasabi leaf extract, which is a natural extract, as an active ingredient.

### [Background Art]

Obesity is a cause of various fatal metabolic diseases, such as cardiovascular diseases, hypertension, and type II diabetes, and in obesity, adipocytes not only store lipids as a long-term energy source but also induce chronic inflammation by secreting various adipokines involved in the metabolism and inflammation of adipocytes and non-adipocytes, thereby causing related metabolic diseases such as insulin resistance. Recently, the incidence of metabolic syndrome, known as type II diabetes, has been increasing globally due to obesity caused by a high-calorie diet and lack of exercise, and it is predicted that, by 2025, the number of patients with metabolic syndrome will double from the current number, reaching approximately 300 million. Obesity caused by abnormal localized fat accumulation, such as abdominal obesity, provides various factors that can seriously induce atheromatous disorders, such as hypertension, hyperlipidemia, blood coagulation disorders, vascular inflammation, and insulin resistance accompanied by abnormally increased insulin secretion, and ultimately contributes to an increased prevalence of fatal cardiovascular diseases. Excessive intake of fatty acids induces the accumulation of triglycerides (TG) in various tissues, accompanied by an increase in lipolysis and resulting in increased circulating fatty acids in the blood, and induces insulin resistance in adipocytes, thereby ultimately causing fat accumulation in non-adipocytes such as muscles, the pancreas, and the liver. When insulin resistance is induced in adipocytes, excess fatty acid-binding and transport proteins cause increased fatty acid uptake by non-adipocytes, and particularly in muscle cells, adversely affect insulin-mediated glucose metabolism. Simultaneously, in the pancreas, prolonged exposure to free fatty acids leads to a vicious cycle in which impaired insulin secretion is induced by the so-called lipotoxicity mechanism. In this case, in the liver as well, high concentrations of free fatty acids accumulate, resulting in insulin resistance and causing large amounts of glucose to begin to be released from the liver. Accumulation of TG in hepatocytes also induces non-alcoholic fatty liver disease (NAFLD), and causes fat accumulation in hepatocytes mainly responsible for glucose metabolism, thereby secondarily inducing steatohepatitis and ultimately inducing fibrosis due to necrosis of hepatocytes. Therefore, the balance between lipid synthesis and degradation in hepatocytes has become an important therapeutic target capable of suppressing insulin resistance and the development of NAFLD associated with metabolic syndrome.

Various therapeutic agents for metabolic syndrome have currently been developed, and in recent years, as appropriate control of oxidative stress, which has been identified as a major cause of diabetes and related complications, along with proper blood glucose regulation, has emerged as a key approach for the treatment of diabetes, attempts have been made to develop α-glucosidase inhibitors or antioxidants with lower side effects and enhanced efficacy.

Among these, metformin has been well known as an AMPK activator, which is a representative oral biguanide-based antidiabetic agent. Metformin is the first-line drug used to treat overweight and obese patients with type II diabetes, who have normal renal function, and is known to be the only drug capable of reducing the incidence of cardiovascular diseases, which is one of the serious complications of diabetes, and regulating the secretion of pancreatic zymogen granules directly involved in lipid digestion and degradation as well as the activity of enzymes related to hepatic glucose metabolism. Metformin is currently known to be the most widely prescribed antidiabetic agent worldwide. Although metformin is mainly used as a drug for treating type II diabetes, prescriptions for polycystic ovary syndrome and premature puberty have also been increasing. Meanwhile, side effects of metformin, such as lactic acidosis due to renal impairment, are also well known.

Accordingly, there is an increasing need for the development of alternative health functional foods and pharmaceuticals derived from natural substances with lower side effects and enhanced efficacy.

Throughout the specification, a number of references are cited and citations thereof are indicated. The disclosures of the cited references are incorporated herein by reference in their entirety to more clearly describe the level of the technical field to which the present invention pertains and the contents of the present invention.

### [Disclosure]

### [Technical Problem]

An object of the present inventors is to provide a composition for preventing, improving, or treating obesity, comprising a wasabi leaf extract as an active ingredient.

Another object of the present inventors is to provide a method for preparing a wasabi leaf extract exhibiting an effect of preventing, improving, or treating obesity.

Yet another object of the present inventors is to provide a method for preventing, improving, or treating obesity using a wasabi leaf extract as an active ingredient.

Further objects and advantages of the present invention will become more apparent from the following detailed description of the invention, claims, and drawings.

### [Technical Solution]

One aspect of the present invention is to provide a composition for preventing, improving, or treating obesity, comprising a wasabi leaf extract as an active ingredient.

Wasabi or Japanese horseradish (Wasabia / Eutrema japonica Matsum.) is a plant native to Japan that has long been used for medicinal purposes, and is a plant that has been used as a spice or pickle because it contains a large amount of isothiocyanate-based compounds such as allylisothiocyanate and thus exhibits a distinctive pungent taste. Although antioxidant and anticancer effects are well known, biological activities of parts other than the wasabi root, particularly the leaf part, are not well known.

The wasabi leaf extract of the present invention comprises isovitexin in an amount of 0.8 to 1.6 mg/g and sinigrin in an amount of 4.8 to 0.6 mg/g, and was confirmed to have at least one effect selected from the group consisting of body weight reduction, body fat reduction, and abdominal fat reduction without causing a decrease in appetite.

The composition of the present invention comprises extracts of the natural components as an active ingredient, and the term "extract" as used herein has a meaning including an extraction product obtained by pressing a raw material or treating a raw material with an extraction solvent, or a processed product obtained by formulating (for example, powdering) the extraction product.

When the extract used in the composition of the present invention is obtained by treating a raw material with an extraction solvent, various extraction solvents may be used, and for example, a polar solvent or a non-polar solvent may be used. As the polar solvent, (i) water, (ii) alcohol (preferably methanol, ethanol, propanol, butanol, n-propanol, iso-propanol, n-butanol, 1-pentanol, 2-butoxyethanol, or ethylene glycol), (iii) acetic acid, (iv) DMFO (dimethyl-formamide), (v) DMSO (dimethyl sulfoxide), and the like may be used, and as the non-polar solvent, acetone, acetonitrile, ethyl acetate, methyl acetate, fluoroalkane, pentane, hexane, 2,2,4-trimethylpentane, decane, cyclohexane, cyclopentane, diisobutylene, 1-pentene, 1-chlorobutane, 1-chloropentane, o-xylene, diisopropyl ether, 2-chloropropane, toluene, 1-chloropropane, chlorobenzene, benzene, diethyl ether, diethyl sulfide, chloroform, dichloromethane, 1,2-dichloroethane, aniline, diethylamine, ether, carbon tetrachloride, THF, and the like may also be used.

Preferably, the extract used in the present invention may be obtained by pressing a raw material or by extraction using any one selected from the group consisting of water, lower alcohols having 1 to 4 carbon atoms, and mixtures thereof as an extraction solvent, but is not limited thereto.

Additionally, the term "extract" as used herein has the meaning commonly used in the art as a crude extract as described above, but broadly also includes a fraction obtained by further fractionating the extract. That is, the extract includes not only an extract obtained by pressing a raw material or using the above-described extraction solvent, but also a product obtained by additionally applying a purification process thereto. For example, a fraction obtained by passing the extract through an ultrafiltration membrane having a predetermined molecular weight cut-off value, a fraction obtained through various additionally performed purification methods such as separation by various chromatography methods (prepared for separation according to size, charge, hydrophobicity, or affinity), and the like are also included in the extract of the present invention.

Additionally, the extract of the present invention may be one from which the solvent has been removed by performing a subsequent additional process, for example, filtering, concentrating, or drying, or may be one that has been subjected to all of filtering, concentration, and drying. For filtration, for example, filter paper or a vacuum filter may be used, for concentration, a vacuum concentrator may be used, and for drying, spray drying, freeze-drying, or the like may be performed to obtain an extract in a powder state.

In one embodiment, the wasabi leaf extract of the present invention may be obtained by extraction using water as an extraction solvent under conditions of a temperature of 98 to 102 °C and a pressure of 0.3 to 0.5 kgf/cm². More specifically, the wasabi leaf extract of the present invention is extracted by grinding a raw material, and then using water in an amount 15 to 20 times the amount of the raw material as an extraction solvent, at 98 to 102 °C for 8±1 hours while maintaining a pressure of 0.3 to 0.5 kgf/cm². The extract solution obtained after completion of extraction may be passed through a filter and then concentrated. The concentration may be performed at a concentration temperature of 60 to 65 °C, and concentration may be performed to a concentration of 30 to 50 brix. The concentrate may be prepared into a powder by performing spray drying.

In the composition of the present invention, the wasabi leaf extract, which is the active ingredient, comprises 0.8 to 1.6 mg/g of isovitexin and 4.8 to 0.6 mg/g of sinigrin, and the weight ratio of isovitexin to sinigrin in the wasabi leaf extract may be 1:1 to 1:6 (isovitexin:sinigrin). Preferably, the weight ratio of isovitexin to sinigrin may be 1:3 to 1:6 (isovitexin:sinigrin) or 1:3±0.5 (isovitexin:sinigrin). When the wasabi leaf extract comprises isovitexin and sinigrin at the above-mentioned weight ratio, the wasabi leaf extract is characterized by exhibiting a more remarkable synergistic effect with respect to efficacy in preventing, improving, and/or treating obesity, as compared with a case in which the wasabi leaf extract comprises isovitexin and sinigrin at a different weight ratio.

The active ingredient may be included in an amount of 0.1 to 5 vol% based on the total volume of the composition of the present invention, and the prevention, improvement, or treatment of obesity may be achieved by a complex mechanism through at least one activity selected from the group consisting of body weight reduction, body fat reduction, and abdominal fat reduction.

In one embodiment, the composition of the present invention may be a pharmaceutical composition. The pharmaceutical composition comprises the above-described active ingredient, and may be formulated into a pharmaceutical unit dosage form by adding a pharmaceutically acceptable carrier, excipient, diluent, and the like.

The term "pharmaceutically acceptable" refers to a non-toxic composition that is physiologically acceptable, does not inhibit the action of an active ingredient when administered to a human, and generally does not cause allergic reactions such as gastrointestinal disorder, dizziness, or similar reactions.

Examples of the carrier, excipient, or diluent may include lactose, dextrose, sucrose, sorbitol, mannitol, xylitol, erythritol, maltitol, starch, acacia gum, alginate, gelatin, calcium phosphate, calcium silicate, cellulose, methyl cellulose, polyvinylpyrrolidone, water, methyl hydroxybenzoate, propyl hydroxybenzoate, talc, magnesium stearate, mineral oil, and the like. Additionally, the pharmaceutical composition may further comprise a filler, an anti-aggregating agent, a lubricant, a wetting agent, a flavoring agent, an emulsifier, a preservative, and the like.

The term "pharmaceutically effective amount" refers to an amount that exhibits a response greater than that of a negative control group, and preferably refers to an amount sufficient to exhibit an effect of preventing and/or treating obesity.

Additionally, the pharmaceutical composition of the present invention may be formulated using methods known in the art so as to provide rapid, sustained, or delayed release of an active ingredient after administration to a mammal. The formulation may be, for example, a formulation selected from the group consisting of gels, pastes, ointments, powders, emulsions, sprays, and aerosols, but is not limited thereto.

Additionally, the pharmaceutical composition of the present invention may be administered in combination with a known compound having an effect of preventing, improving, and/or treating obesity.

Additionally, the pharmaceutical composition of the present invention may be formulated using methods known in the art so as to provide rapid, sustained, or delayed release of an active ingredient after administration to a mammal. The formulation may be in the form of a powder, granule, tablet, emulsion, syrup, aerosol, soft or hard gelatin capsule, sterile injectable solution, or sterile powder.

The route of administration of the pharmaceutical composition of the present invention may be oral or parenteral, but is not limited thereto. The parenteral route of administration may include various routes such as, for example, transdermal, nasal, intraperitoneal, intramuscular, subcutaneous, or intravenous routes. Additionally, the pharmaceutical composition of the present invention may be administered in combination with a known compound having an effect of preventing and/or treating metabolic syndrome.

In another embodiment, the composition of the present invention may be a food composition.

The food composition of the present invention includes all processed forms of natural materials, such as foods, functional foods, nutritional supplements, health feed, and food additives. The food composition of the type may be prepared in various forms according to conventional methods known in the art.

For example, as a health food, the extract of the present invention itself may be prepared in the form of tea, juice, or drink for drinking, or may be granulated, encapsulated, or powdered for ingestion. Additionally, in addition to the extract of the present invention, white peony, corni fructus, Acanthopanax, Ganoderma lucidum, citrus peel, Eucommia bark, Angelica gigas, gardenia, Astragalus, malt, trifoliate orange, vitamin C, fructooligosaccharide, stevioside, purified water, maltodextrin, and the like may be further included alone or in combination within a range that does not interfere with the purpose of the present invention, but other pharmaceutically active ingredients and/or additives that may be further included in the food composition of the present invention are not limited to the above examples.

For example, the food composition according to the present invention may comprise thiamine (vitamin B1), riboflavin, ascorbic acid, niacin, and vitamin B6 as water-soluble vitamins, may comprise myristic acid, palmitic acid, stearic acid, oleic acid, linoleic acid, and the like as fatty acids, may comprise glycolic acid and acetic acid as weak acid components, and may comprise, as amino acids, eight essential amino acids including threonine, valine, methionine, isoleucine, leucine, phenylalanine, tryptophan, and lysine, as well as aspartic acid, serine, glutamic acid, proline, glycine, alanine, cysteine, tyrosine, histidine, arginine, and the like.

The composition of the present invention may be used as an additive or therapeutic agent for the purpose of improving obesity in animals other than humans as well as humans. Thus, the present invention provides a composition for preventing, improving, or treating animal obesity, a feed additive for preventing or improving animal obesity comprising the above-described composition, and a therapeutic agent for animal obesity comprising the above-described composition.

Another example of the present invention provides a method for preparing a wasabi leaf extract for preventing, improving, or treating obesity.

The extract may be obtained by mixing wasabi leaves with an extraction solvent and extracting the mixture at a temperature of 96 to 110 °C and a pressure of 0.3 to 0.7 kgf/cm².

The wasabi leaves may preferably be dried leaves.

The wasabi leaves may preferably be dried leaves.

In one embodiment of the present invention, provided is a use of a wasabi leaf extract or a composition comprising a wasabi leaf extract for use in the manufacture of a food, drug, or health functional food for treating, preventing, or improving obesity. The wasabi leaf extract comprises 0.8 to 1.6 mg/g of isovitexin and 4.8 to 0.6 mg/g of sinigrin, and the weight ratio of isovitexin to sinigrin in the wasabi leaf extract may be 1:1 to 1:6 (isovitexin:sinigrin). In order to include an active ingredient in the extract at an optimal content, wasabi leaves may be mixed with an extraction solvent and extracted at a temperature of 96 to 110 °C and a pressure of 0.3 to 0.7 kgf/cm².

### [Advantageous Effects]

The composition of the present invention exhibits at least one effect selected from the group consisting of body weight reduction, body fat reduction, and abdominal fat reduction without causing a decrease in appetite, thereby exhibiting an effect of preventing, improving, or treating obesity.

### [Description of Drawings]

FIG. 1 shows a photograph in which a wasabi leaf extract was obtained as deep brown powders according to one embodiment.
FIGS. 2A-2C show results of analyzing the contents of standard materials, including isovitexin, in the wasabi leaf extract by an HPLC method.
FIG. 3 shows results of comparatively confirming intracellular lipid accumulation in the cells of each group through an Oil Red O test after removal of the culture medium.
FIG. 4 shows results of confirming changes in body weight over time after administration of a material in experimental animals of each group.
FIG. 5 shows results of confirming body weight gain at 6 days and 84 days after administration of a material in experimental animals of each group.

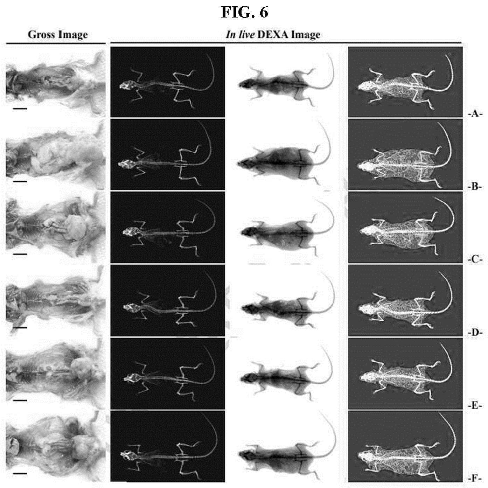

FIG. 6 shows representative whole-body DEXA (Dual-energy X-ray absorptionmetry) images showing total body mass and abdominal fat pads at 84 days after administration of a material in experimental animals of each group. (A = mouse fed NFD and orally administered vehicle (distilled water) at 10 mL/kg (intact control); B = mouse fed HFD and orally administered vehicle at 10 mL/kg (HFD control); C = HFD-fed mouse orally administered metformin at 250 mg/kg (metformin); D = HFD-fed mouse orally administered WL at 200 mg/kg (WL200); E = HFD-fed mouse orally administered WL at 100 mg/kg (WL100); F = HFD-fed mouse orally administered WL at 50 mg/kg (WL50))
FIG. 7 is a graph showing changes in whole-body and abdominal fat density at 84 days after administration of a material in experimental animals of each group.

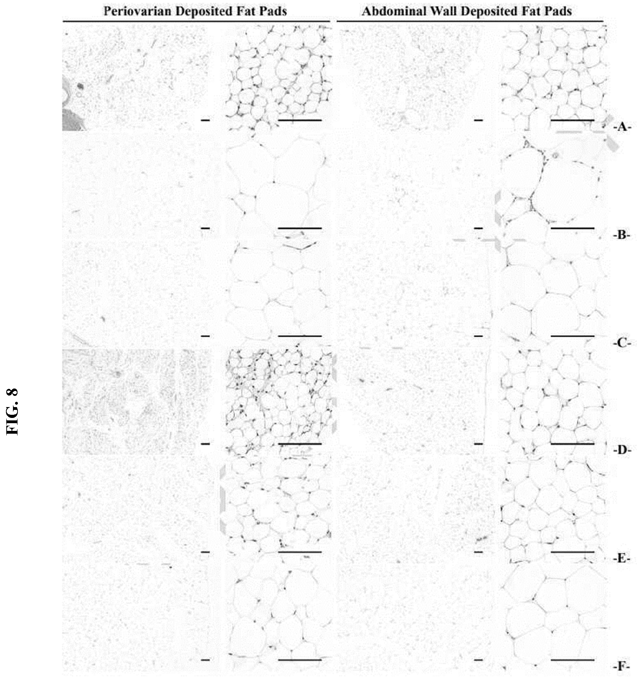

FIG. 8 shows representative histological images of adipocytes taken from mouse periovarian and abdominal wall deposited fat pads at 84 days after administration of a material in experimental animals of each group. (A = mouse fed NFD and orally administered vehicle (distilled water) at 10 mL/kg (intact control); B = mouse fed HFD and orally administered vehicle at 10 mL/kg (HFD control); C = HFD-fed mouse orally administered metformin at 250 mg/kg (metformin); D = HFD-fed mouse orally administered WL at 200 mg/kg (WL200); E = HFD-fed mouse orally administered WL at 100 mg/kg (WL100); F = HFD-fed mouse orally administered WL at 50 mg/kg (WL50))

### [Modes of the Invention]

Hereinafter, the present invention will be described in further detail through examples. These examples are provided only to describe the present invention in more detail, and it will be apparent to those of ordinary skill in the art to which the present invention pertains that the scope of the present invention is not limited by these examples.

### Examples

### I. Preparation and analysis of extracts

### 1. Preparation of wasabi leaf extracts (Folium, Wasabia japonica(Miq.) Matsum extracts)

It was confirmed that, during the preparation of wasabi leaf extracts, the contents of isovitexin and sinigrin, which are active ingredients of wasabi leaf, in the extract varied depending on the temperature and pressure conditions. Accordingly, in order to determine the optimal extraction conditions for isovitexin and sinigrin, which are active ingredients of wasabi leaf, when water was used as a solvent, extraction was performed under different temperature and pressure conditions, and the results are shown in Table 1 below.

**[Table 1]**

| **Extraction condition (temperature, pressure (kgf/cm²))** | **Isovitexin (mg/g)** | **Sinigrin (mg/g)** | **Isovitexin:sinigrin ratio** |
|---|---|---|---|
| 115~125 °C, 1.5 | 0.25 | 3.2 | 1:12.8 |
| 98~102 °C, 0.3 to 0.5 | 1.35 | 6.8 | 1:5.03 |
| 95 °C, 0.25 to 0.3 | 0.8 | 4.2 | 1:5.25 |

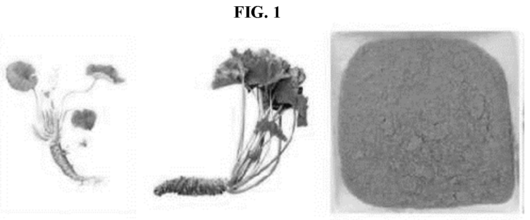

The experimental results confirmed that the active ingredient content of the wasabi leaf extract exhibiting the optimal effect was 0.8 to 1.6 mg/g of isovitexin and 4.8 to 9.6 mg/g of sinigrin, and experiments were conducted with three different temperatures under conditions for producing raw materials satisfying these criteria, thereby confirming the extraction of approximately 80% of isovitexin and sinigrin at 95 °C or less as shown in Table 1, and confirming that the optimal contents and proportions were achieved under a temperature condition of 98 to 102 °C. In addition, it was confirmed that, when extraction was performed at 115 to 125 °C, the active ingredients were degraded by heat, resulting in a drastic decrease in their contents. Therefore, it was confirmed that the optimal extraction condition for extracting isovitexin and sinigrin, which are active ingredients of the wasabi leaf extract, was 98 to 102 °C. Accordingly, the wasabi leaf extract was prepared as follows. Extraction was performed by adding 18 times the amount of water relative to the raw material to 150 kg of dry wasabi leaves, and maintaining a temperature of 100 °C for 8 hours under a pressure of 0.4 to 0.5 kgf/cm², with a temperature variation of ±0.5 °C. The extract that was achieved by extraction was passed through a 1 µm filter, transferred to a buffer tank, and subsequently transferred in appropriate amounts to a concentration tank. The concentration temperature was 60 to 65 °C, and concentration was performed until a concentration of 30 to 50 brix. The concentrate was mixed with dextrin at a proportion of 0 to 10% to prepare a wasabi leaf extract as a deep brown powder using a spray dryer at an inlet temperature of 170 to 200 °C and an outlet temperature of 85 to 100 °C (FIG. 1).

### 2. Confirmation of active ingredient contents in wasabi leaf extract

The contents of the active ingredients included in the wasabi leaf extract were analyzed as follows.

### (1) Preparation of standard solution

Standard material 1: isovitexin
Molecular formula: C₂₁H₂₀O₁₀, Molecular weight: 432.38
CAS No. : 38953-85-4
Standard material 2: sinigrin
Molecular formula: C₁₀H₁₇NO₉S₂, Molecular weight: 359.36
CAS No. : 534-69-0

Approximately 8 mg of the standard material was accurately weighed and dissolved in methanol to an exact volume of 50 mL, thereby preparing a standard stock solution. 3 mL of the standard stock solution was diluted with methanol to 50 mL, and the resulting solution was used as a standard solution.

### (2) Preparation of test solution

Approximately 330 mg of the wasabi leaf extract was accurately weighed, methanol was added thereto, and ultrasonic extraction was performed for 30 minutes. Thereafter, the volume was adjusted to an exact volume of 50 mL with methanol, and the resulting solution was used as a test solution. The test solution was filtered through a 0.45-µm syringe filter, placed in a brown vial, and subjected to analysis.

### (3) Analysis

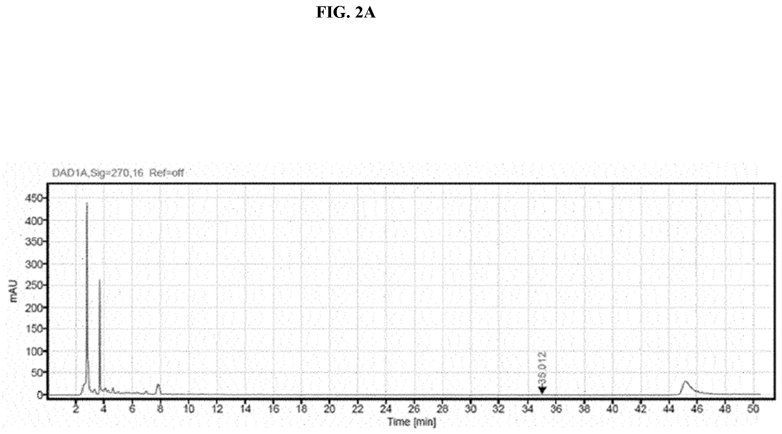

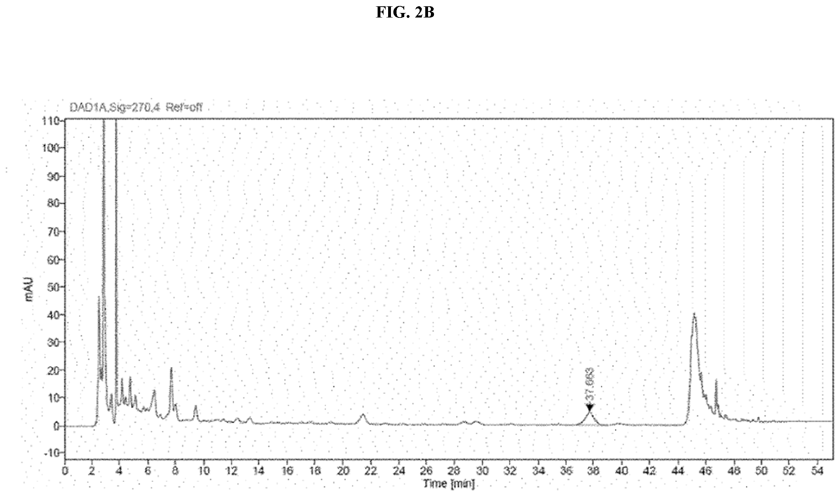

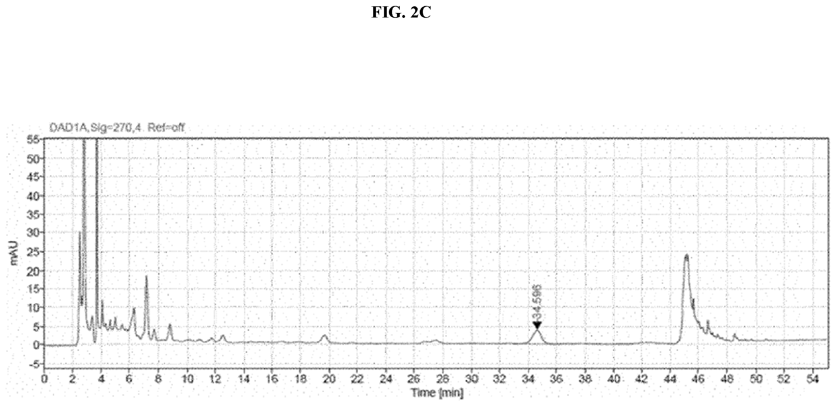

High speed liquid chromatography (HPLC) analysis was performed under the HPLC conditions shown in Table 2 below and the mobile phase conditions shown in Table 3 below, and the results are shown in FIG. 2.

**[Table 2]**

| **Item** | **Condition** |
|---|---|
| Injection volume | 10 µL |
| Column temperature | 30 °C |
| Mobile phase | A: water with 0.05% TFA |
| | B: acetonitrile |
| Flow rate | 1.0 mL/min |
| Detection wavelength | 270 nm |

**[Table 3]**

| Time (min) | Mobile phase | B |
|---|---|---|
| Initial | 86 | 14 |
| 40 | 85 | 15 |
| 45 | 5 | 95 |
| 55 | 5 | 95 |

### (4) Calculation

A calibration curve was established based on the peak area of the standard solution, and the concentration of the active ingredient in the test solution was calculated using the calibration curve equation, followed by calculation of the active ingredient contents using the following equation. $Active ingredient content \left(mg/g\right) = \left(A\times B\times C\times D\right) / E$
A: Concentration of test solution (µg/mL)
B: Final volume (mL)
C: Dilution factor
D: Purity of reference standard (%)
E: Sample amount (mg)

As a result of the experiment, it was confirmed that the wasabi leaf extract prepared in 1 contained 0.8 to 1.6 mg/g of isovitexin and 4.8 to 9.6 mg/g of sinigrin.

### 3. Evaluation of efficacy according to the ratio of active ingredient contents

To evaluate efficacy according to the optimal blending ratio of isovitexin and sinigrin, which are active ingredients of wasabi leaf, 3T3-L1 preadipocyte cells purchased from ATCC were subcultured in DMEM medium (containing 10% bovine serum, 100 units/mL penicillin, and 100 µg/mL streptomycin) to induce adipocyte differentiation and adipogenesis. As a normal group, preadipocyte cells were used, and as a control group, preadipocyte cells cultured to a post-confluent state after seeding were induced to differentiate, followed by replacement of the medium once every two days to maintain adipocytes. Isovitexin and sinigrin, which are the active ingredients of the wasabi leaf extract, were each treated at 100 µg/mL, and mixtures thereof at blending ratios of isovitexin : sinigrin = 6:1, 3:1, 1:1, 1:3, 1:6, and 1:9 were added and treated at the same concentration of 100 µg/mL, respectively.

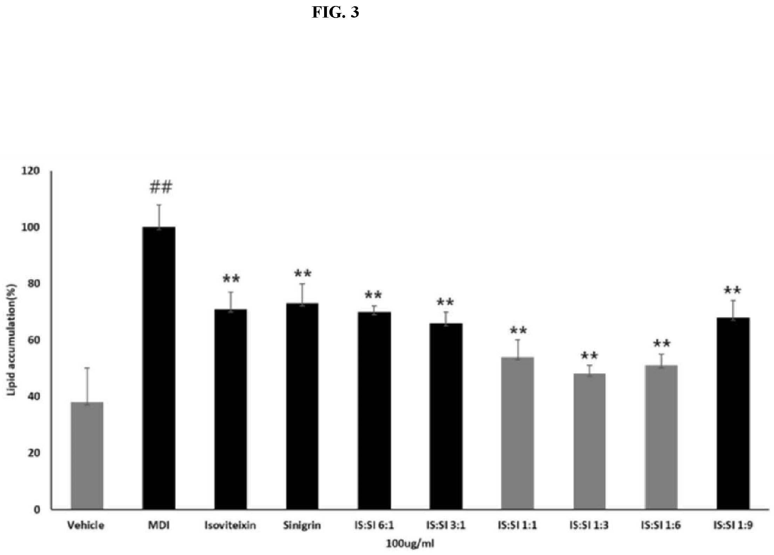

To confirm the effect on fat accumulation according to the optimal ratio of active ingredients in wasabi extract, the cells of each group were subjected to an Oil Red O test after removal of the culture medium to comparatively evaluate intracellular lipid accumulation, and the results are shown in FIG. 3.

The amount of intracellular lipid accumulation in cells included in the positive induction control group, in which differentiation was induced by MDI (3-isobutyl-1-methylxanthine, dexamethasone, and insulin), was set to 100%, and the relative amount of intracellular lipid accumulation according to each component was expressed as a percentage (%).

As a result of the experiment, when the vehicle, MDI, isovitexin alone, sinigrin alone, an isovitexin (IS):sinigrin (SI) 6:1 mixture, an IS:SI 3:1 mixture, an IS:SI 1:1 mixture, an IS:SI 1:3 mixture, an IS:SI 1:6 mixture, and an IS:SI 1:9 mixture were each treated at the same concentration of 100 µg/mL, the fat accumulation ratios were confirmed to be 38%, 100%, 71%, 73%, 70%, 66%, 54%, 48%, 51%, and 68%, respectively, and it was confirmed that the mixtures exhibited a superior inhibitory effect on fat accumulation at all ratios as compared with treatment with isovitexin alone or sinigrin alone. Particularly, in the IS:SI 1:1 mixture, 1:3 mixture, and 1:6 mixture, it was confirmed that fat accumulation was further inhibited by 17%, 23%, and 20%, respectively, as compared with the single-treatment group. Accordingly, it was confirmed that, in the wasabi leaf extract, when the content ratio of isovitexin and sinigrin was present at 1:1 to 1:6, preferably, at 1:3 to 1:6, and most preferably, at 1:3, superior fat accumulation inhibitory activity was exhibited.

Thus, subsequently, an additional *in vivo* efficacy evaluation was performed using the 1:3 mixture (the composition including a wasabi extract in which isovitexin and sinigrin are combined at a weight ratio of 1:3 (IS: SI) (hereinafter, WL), which was confirmed to have the most remarkable effect in fat accumulation inhibitory activity.

### II. Evaluation of in vivo efficacy

### Experimental animals and methods

A total of 165 6-week-old female SPF/VAF CrljOri:CD1[ICR] mice [OrientBio, Seongnam, Korea] were acclimated to the laboratory environment for 10 days and then adapted to a high fat diet (HFD) for 1 week, after which only experimental animals exhibiting a consistent body weight increase were divided into a total of 6 groups with 8 animals per group and used in the experiment. All experimental animals were handled under prior approval of the Institutional Animal Care and Use Committee of Daegu Haany University.

### Group division (total: 6 groups; 8 mice per group):

Normal control group (Normal pellet diet; NFD-fed vehicle-treated group)
HFD control group (45%Kcal high fat diet; HFD-fed vehicle-treated group)
HFD-fed control drug group treated with metformin 250 mg/kg
HFD-fed experimental group treated with the highest dose of WL (200 mg/kg)
HFD-fed experimental group treated with the medium dose of WL (100 mg/kg)
HFD-fed experimental group treated with the low dose of WL (50 mg/kg)

### Dose Frequency

Administration frequency: Once per day, 84 days
Administration route: Oral administration
Vehicle: Sterile distilled water
Administration dose: 10 mL/kg

### Determination of administration dose of experimental material:

In the present experiment, the highest dose of WL was determined to be 200 mg/kg based on a previous pharmacological efficacy screening experiment conducted by the present researchers using a 45% kcal HFD-fed mouse model, and the medium and low doses were set at 100 and 50 mg/kg, respectively, at a common ratio of 2. The WL was dissolved in sterile distilled water. Forced oral administration of the resulting WL solution was performed at 10 mL/kg, which is the general oral administration volume for mice [Flecknell, 1996; KFDA Guidelines, Notification No. 2017-071, 2017]. In addition, the administration dose of metformin used as the control drug was determined based on previous references and was set at 250 mg/kg [Jeong et al., 2013; Choi et al., 2017]. Metformin is a representative drug that is used as an anti-obesity agent through AMPK activation.

### Administration of experimental material:

After a 1-week adaptation period to HFD, an appropriate amount of WL was dissolved in sterile distilled water, and forced oral administration was performed at a dose of 10 mL/kg using a 1-mL syringe equipped with a metal zonde. In other words, WL was dissolved in distilled water at concentrations of 20, 10, and 5 mg/mL, respectively, and was orally administered once daily for 84 days at a dose of 10 mL/kg (200, 100, and 50 mg/kg). In addition, metformin was also dissolved in sterile distilled water at a concentration of 25 mg/mL, and then orally administered once daily for 84 days at a dose of 10 mL/kg (250 mg/kg). In the normal and HFD control groups, to administer in the same manner and alleviate stress, only sterile distilled water instead of the test material was administered by forced oral administration for the same period of time.

### Experimental results

### 1. Change in body weight

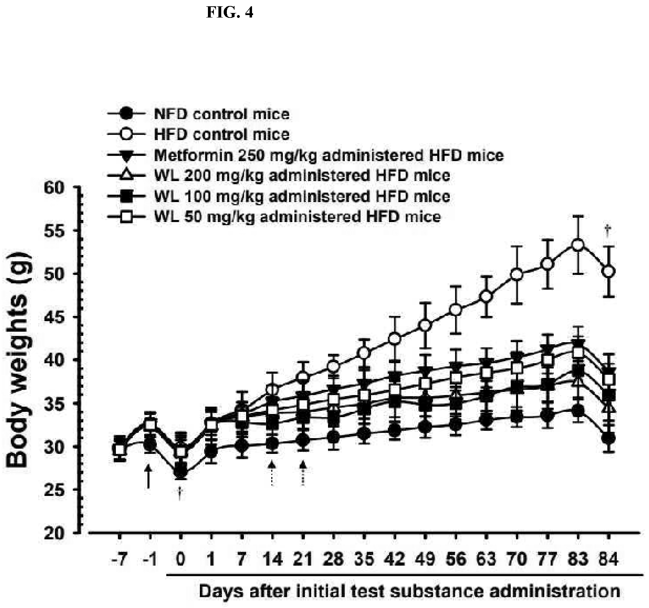

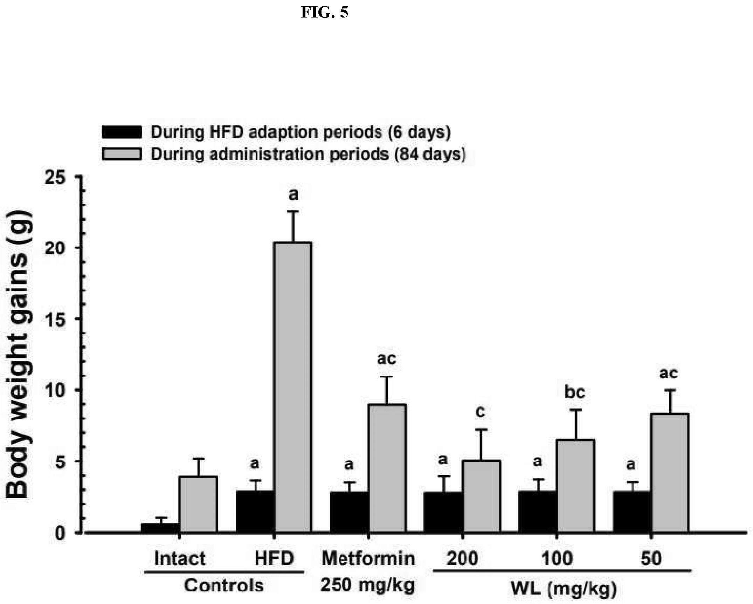

Since only experimental animals exhibiting a consistent body weight increase were selected after 1 week of HFD feeding, in the HFD control group, a significant (p<0.01) increase in body weight as compared with the normal control group was observed from 6 days after the initiation of high-fat diet feeding. The body weight gains during the 1-week HFD adaptation period and the 84-day test material administration period also showed significant (p<0.01) increases compared with the normal control group. Meanwhile, a significant (p<0.01 or p<0.05) decrease in body weight compared with the HFD control group began to be observed from day 14 after the initiation of administration in all three 200, 100, and 50 mg/kg WL administration groups, and from day 21 in the metformin 250 mg/kg administration group. Furthermore, a significant decrease (p<0.01) in body weight gain during the 84-day test substance administration period was observed in all test material administration groups compared with the HFD control group. Particularly, the WL 50 mg/kg-treated group exhibited an inhibitory effect on increases in HFD-fed body weight and body weight gain, compared with that of the metformin 250 mg/kg-treated group (Table 4, and FIGS. 4 and 5).

**[Table 4]**

| | Times | Body weights (g) at days after initial test substance treatment | | | | Body weight gains during | | Mean Daily Food Consumption (g) |
|---|---|---|---|---|---|---|---|---|
| Groups | | 7 days before [A] | 1 day before [B] | 0 day* [C] | 84 days* [D] | Adapt period [B-A] | Administration period [D-C] | |
| Controls | | | | | | | | |
| | Intact | 29.83±1.05 | 30.19±0.93 | 27.03±0.81 | 30.94±1.56 | 0.56±0.48 | 3.91±1.29 | 6.07±0.25 |
| | HFD | 29.79±1.41 | 32.66±1.34 | 29.81±1.70 | 50.23±2.91 | 2.88±0.77 | 20.41±2.10 | 5.04±0.15 |

| Reference | | | | | | | | |
|---|---|---|---|---|---|---|---|---|
| | Metformin | 29.66±1.24 | 32.48±1.42 | 29.66±1.98 | 38.63±2.02 | 2.81±0.70 | 9.96±1.98 | 5.06±0.22 |

| Test materials - WL | | | | | | | | |
|---|---|---|---|---|---|---|---|---|
| | 200 mg/kg | 29.50±1.16 | 32.29±1.61 | 29.36±1.91 | 34.43±2.81 | 2.79±1.17 | 5.06±2.14 | 5.03±0.20 |
| | 100 mg/kg | 29,66±1.33 | 32.53±1.39 | 29.46±1.54 | 35.95±2.92 | 2.86±0.86 | 6.49±2.15 | 5.04±0.35 |
| | 50 mg/kg | 29.65±1.15 | 32.50±1.32 | 29.40±1.34 | 37.76±1.79 | 2.85±0.68 | 8.36±1.62 | 5.08±0.28 |

With respect to the body weight gain during the 84-day administration period, the HFD control group showed a change of 421.73% compared with the normal control group, whereas the metformin 250 mg/kg-, WL 200, 100, and 50 mg/kg-treated groups showed changes of - 56.09, -75.20, -68.22, and -59.03%, respectively, compared with the HFD control group.

### 2. Changes in mean feed intake

Although a significant (p<0.01) decrease in mean feed intake was observed in the HFD control group compared with the normal control group, no significant change in mean feed intake relative to the HFD control group was observed in any of the test material-treated groups, including the metformin 250 mg/kg-treated group (Table 4). In terms of mean feed intake, the HFD control group showed a -16.99% change compared with the normal control group, whereas the metformin 250 mg/kg-, WL 200, 100, and 50 mg/kg-treated groups showed changes of 0.32, -0.14, -0.04, and 0.71%, respectively, compared with the HFD control group.

### 3. Changes in body and abdominal fat mass

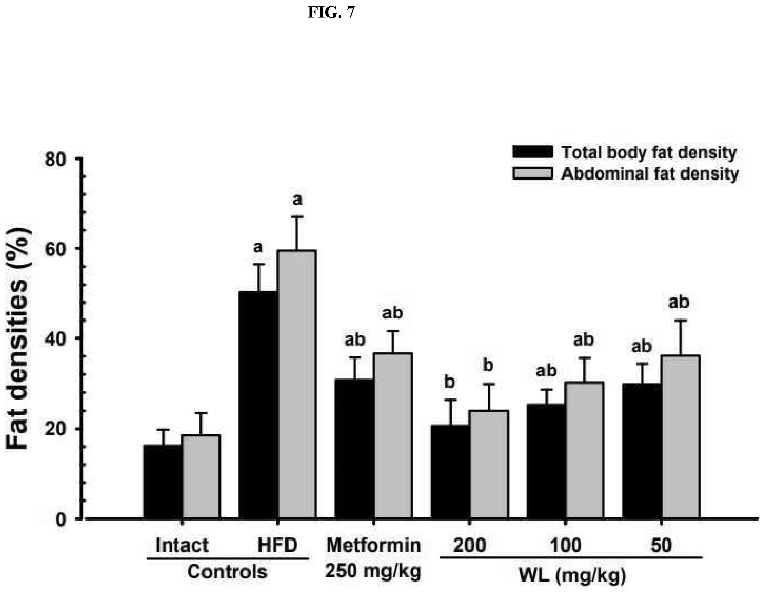

Although the HFD control group showed significant (p<0.01) increases in body and abdominal fat mass, respectively, compared with the normal control group, the WL-treated groups at all three doses exhibited significant dose-dependent (p<0.01) decreases in body and abdominal fat mass compared to the HFD control group. Particularly, the WL 50 mg/kg-treated group showed an inhibitory effect on HFD-induced increases in body and abdominal fat mass comparable to those of the metformin 250 mg/kg-treated group (FIGS. 6 and 7).

Regarding body fat mass, the HFD control group showed a 210.56% change compared with the normal control group, whereas the metformin 250 mg/kg-, WL 200, 100, and 50 mg/kg-treated groups showed changes of -38.91, -59.03, -49.78, and -40.70%, respectively, compared with the HFD control group. Regarding abdominal fat mass, the HFD control group showed a 221.25% change compared to the normal control group, whereas the metformin 250 mg/kg-, WL 200, 100, and 50 mg/kg-treated groups showed changes of -38.33, -59.65, - 49.18, and -39.24%, respectively, compared with the HFD control group.

### 4. Changes in fat weight

The HFD control group showed significant (p<0.01) increases in relative and absolute weights of periovarian and abdominal wall fat deposits compared with the normal control group, whereas the WL-treated groups at all three doses exhibited significant (p<0.01) dose-dependent decreases in periovarian and abdominal wall fat deposits compared with the HFD control group. Particularly, the WL 50 mg/kg-treated group showed an inhibitory effect on HFD-induced increases in the relative and absolute weights of periovarian and abdominal wall fat deposits, which was comparable to that of the metformin 250 mg/kg-treated group (FIG. 6).

The HFD control group showed changes of 916.67 and 528.77% in the relative and absolute weights of periovarian fat deposits, respectively, compared with the normal control, whereas the metformin 250 mg/kg-, WL 200, 100, and 50 mg/kg-treated groups showed changes of -61.56, -80.90, -70.07, and -61.85%, respectively, in the absolute weight of periovarian fat deposits, and changes of -49.83, -72.52, -57.89, and -49.24%, respectively, in the relative weight of periovarian fat deposits compared with the HFD control group. The HFD control group showed changes of 1091.25 and 635.54% in the absolute and relative weights of abdominal wall fat deposits compared with the normal control group, whereas the metformin 250 mg/kg-, WL 200, 100, and 50 mg/kg-treated groups showed changes of -61.28, -77.33, -70.93, and -62.71% in the absolute weight of abdominal wall fat deposits and changes of -49.72, -67.46, -59.71, and -50.42% in the relative weight of abdominal wall fat deposits compared with the HFD control group.

### 5. Histopathological changes in periovarian and abdominal wall fat deposits

In the HFD control group, marked hypertrophy of adipocytes was observed, and significant (p<0.01) increases in the thickness of periovarian and abdominal wall adipose tissues and in adipocyte diameter were observed, respectively, compared to the normal control group. In contrast, the WL-treated groups at all three doses showed significant (p<0.01) dose-dependent decreases in the thicknesses of the periovarian and abdominal wall fat deposits and in adipocyte diameter compared with the HFD control group. Particularly, the WL 50 mg/kg-treated group showed inhibitory effects on HFD-induced increases in the thickness of periovarian and abdominal wall adipose tissues and in adipocyte diameter comparable with those of the metformin 250 mg/kg-treated group (FIG. 8).

The HFD control group showed a 286.18% change in periovarian adipose tissue thickness compared with the normal control group, whereas the metformin 250 mg/kg-, WL 200, 100, and 50 mg/kg-treated groups showed changes of -49.84, -66.48, -61.14, and -51.62% in periovarian adipose tissue thickness, respectively, compared with the HFD control group. Regarding the mean adipocyte diameter of periovarian adipose tissue, the HFD control group showed a 235.21% change compared with the normal control group, whereas the metformin 250 mg/kg-, WL 200, 100, and 50 mg/kg-treated groups showed changes of -39.34, -63.31, - 54.43, and -40.47%, respectively, compared with the HFD control group. Regarding the thickness of abdominal wall adipose tissue, the HFD control group showed a 454.29% change compared with the normal control group, whereas the metformin 250 mg/kg-, WL 200, 100, and 50 mg/kg-treated groups showed changes of -52.59, -73.83, -67.58, and -53.85%, respectively, compared with the HFD control group. Regarding the mean adipocyte diameter of the abdominal wall adipose tissue, the HFD control group showed a 181.53% change compared with the normal control group, whereas the metformin 250 mg/kg-, WL 200, 100, and 50 mg/kg-treated groups showed changes of -36.84, -56.22, -49.75, and -38.48%, respectively, compared with the HFD control group.

### [Industrial Applicability]

The present invention may be provided as a composition for preventing or improving obesity, preferably as a food or health functional food. The present invention may be provided as a drug for treating obesity.

## Claims

1. A composition for preventing, improving, or treating obesity, comprising a wasabi leaf extract,
wherein the wasabi leaf extract comprises 0.8 to 1.6 mg/g of isovitexin and 4.8 to 9.6 mg/g of sinigrin, and the weight ratio of isovitexin to sinigrin in the composition is 1:1 to 1:6 (isovitexin:sinigrin).

2. The composition of claim 1, wherein the extract is obtained using any one selected from the group consisting of water, lower alcohols having 1 to 4 carbon atoms, and mixtures thereof as an extraction solvent.

3. The composition of claim 1, wherein the weight ratio of isovitexin to sinigrin in the composition is 1:3 to 1:6 (isovitexin:sinigrin).

4. The composition of claim 1, wherein the weight ratio of isovitexin to sinigrin in the composition is 1:3±0.5 (isovitexin:sinigrin).

5. The composition of claim 1, wherein the wasabi leaf extract is obtained using water as an extraction solvent under conditions of a temperature of 98 to 102 °C and a pressure of 0.3 to 0.5 kgf/cm².

6. The composition of claim 1, wherein the composition exhibits one or more effects selected from the group consisting of body weight reduction, body fat reduction, and abdominal fat reduction without causing a decrease in appetite.

7. The composition of any one of claims 1 to 6, wherein the composition is for prevention and improvement of obesity, and is a food composition.

8. The composition of any one of claims 1 to 6, wherein the composition is a pharmaceutical composition.

9. A health functional food comprising the composition of claim 7.

10. The composition of claim 1, wherein the composition is for prevention, improvement, or treatment of obesity in mammals.

11. A feed additive for preventing or improving obesity in mammals, comprising the composition of claim 10.

12. A therapeutic agent for obesity in mammals, comprising the composition of claim 10.
